(19) Europäisches Patentamt
European Patent Office
Office européen des brevets

(11) Publication number: **0 111 607**
**B1**

(12) # EUROPEAN PATENT SPECIFICATION

(45) Date of publication of patent specification: **21.05.86**

(51) Int. Cl.$^4$: **C 07 D 451/12,** A 61 K 31/46

(21) Application number: **82402261.0**

(22) Date of filing: **10.12.82**

(54) **Tropyl and pseudotropyl alkyl-benzoates and their use in migraine treatment.**

(43) Date of publication of application:
**27.06.84 Bulletin 84/26**

(45) Publication of the grant of the patent:
**21.05.86 Bulletin 86/21**

(84) Designated Contracting States:
**AT BE CH DE FR IT LI LU NL SE**

(56) References cited:
**JOURNAL OF HETEROCYCLIC CHEMISTRY, vol. 15, March 1978, pages 315-317, Columbus, Ohio, USA, J.L. WALLACE et al.: "The synthesis of several para-substituted tropanyl benzoates"**

**CHEMICAL ABSTRACTS, vol. 59, 1963, column 5665h, Columbus, Ohio, USA, A.L. MNDZHOYAN et al.: "Relation of pharmacological action with chemical structure in a series of tropine esters"**

**The file contains technical information submitted after the application was filed and not included in this specification**

(73) Proprietor: **MERRELL TORAUDE ET COMPAGNIE**
**16 Rue d'Ankara**
**F-67084 Strasbourg (FR)**

(72) Inventor: **Fozard, John Richard**
**22 Rue Vermeer**
**F-67200 Strasbourg-Elsau (FR)**
Inventor: **Gittos, Maurice Ward**
**16 Rue Andre Malraux**
**Plobsheim F-67400 Illkirch-Graffenstaden (FR)**

(74) Representative: **Burford, Anthony Frederick et al**
**W.H. Beck, Greener & Co. 7 Stone Buildings**
**Lincoln's Inn**
**London WC2A 3SZ (GB)**

(56) References cited:
**JOURNAL OF THE CHEMICAL SOCIETY, Perkin Transactions I, 1979, pages 2130-2132, Columbus, Ohio, USA, MOHAMMED A.I. AL-YAHYA et al.: "Alkaloids of the genus Erythroxylum. Part 2"**

**EP 0 111 607 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

The invention relates to the treatment of migraine with certain tropyl and pseudotropyl alkylbenzoates and provides pharmaceutical compositions comprising said compounds. Further, the invention also provides said compounds for use in treating migraine and, when said compounds are novel, it provides said novel compounds *per se*.

Background of the Invention

Acute attacks of migraine are usually treated with a peripheral vasoconstrictor, such as ergotamine, which may be co-administered with caffeine, and dihydroergotamine; an antipyretic analgesic, such as acetylsalicylic acid or p-actylaminophenol; and/or an anti-emetic such as cyclizine, metoclopramide and thiethylperazine. It has also been reported (J. B. Hughes; Med. J. Aust. 2, No. 17, 580, 1977) that immediate relief of acute migraine attack can be obtained by slow intravenous injection of metoclopramide (10 g).

It is believed that 5-hydroxytryptamine (5-HT) is the naturally occurring substance most likely to play a role in the pathophysiology of migraine. Increased amounts of 5-HT and its metabolite 5-hydroxyindoleacetic acid are excreted in the urine during most attacks. Further, plasma and platelet 5-HT concentrations fall rapidly at the onset of an attack and remain low whilst the headache persists. Moreover, attacks of migraine have been clearly associated with periods of thrombocytopaenia in certain patients. It has been proposed that compounds which block the activity of 5-HT would be of use in the treatment of migraine (J. R. Fozard, International Headache Congress 1980) reported in Advances in Neurology, Vol 33, Raven Press, New York 1982).

The known migraine prophylactic drugs methysergide, propranolol, amitriptyline, and chlorpromazine have widely different pharmacological activities but are all 5-HT D-receptor antagonists at the doses used clinically for the treatment of migraine. Metoclopramide is a potent 5-HT M-receptor antagonist and it has been proposed (J. R. Fozard *supra*) that blockade of the M-receptor present on afferent sensory neurones affords symptomatic relief in an acute migraine attack.

The potency as a 5-HT M-receptor antagonists of (−) cocaine and some related compounds, including benzoyl pseudotropine (i.e. pseudotropyl benzoate) and benzoyl tropine (i.e. tropyl benzoate) has been reported (J. R. Fozard *et al*, Eur. J. Pharmacol., 59(1979), 195—210) but, with the exceptions of nor(−)cocaine and benzoyl tropine, none are as potent as metoclopramide. The $pA_2$ values reported for nor(−)cocaine, benzoyl tropine, and benzoyl pseudotropine are 7.7, 7.2 and 7.0 respectively whilst the $pA_2$ 5-HT value determined for metoclopramide by the same procedure is 7.2 (J. R. Fozard *et al.* Eur. J. Pharmacol., 49(1978), 109—112).

It has been reported in European Patent Application No. 82401060.7 (now EP—0067770) that substitution of tropyl benzoate with alkyl, alkoxy or halogen in the 3; or 3 and 5; or 3, 4 and 5 positions of the benzene ring surprisingly enhances its potency as a 5-HT M-receptor antagonist. Tests conducted with tropyl-4-chlorobenzoate ($pA_2$ 7.0), tropyl-4-methylbenzoate ($pA_2$ 7.8), tropyl-3,4-dichlorobenzoate ($pA_2$ 7.8) and tropyl-3,4-dimethoxybenzoate ($pA_2$ 7.2), indicated that corresponding substitutions elsewhere in the benzene ring would not provide the same order of increase in potency. Further, tests conducted with pseudotropyl-3,5-dimethoxybenzoate ($pA_2$ 6.6), and pseudotropyl-3,4,5-trimethoxybenzoate ($pA_2$ 5.7) indicated that corresponding substitutions in the benzene ring of pseudotropyl benzoate actually would reduce its potency as a 5-HT M-receptor antagonist. However, it has now surprisingly been found that substitution by alkyl in an least the 2 posititon or in the 3,4 position of the benzene ring of tropyl benzoate and pseudotropyl benzoate and in the 3, or 3 and 5, or 3, 4 and 5 positions of the benzene ring of pseudotropyl benzoate does substantially enhance potency as a 5-HT M-receptor antagonist.

All the tropyl benzoate derivatives of Formula I except tropyl-2-methylbenzoate, and all the pseudo tropyl benzoates of Formula I are believed to be novel compounds. Tropyl-2-methylbenzoate is disclosed in Al-Yahya *et al* (J. Chem. Soc. Perkins Trans. 1, 1979 (9), 2130—2) but no pharmacological use has been reported for the compound.

Summary of the Invention

According to a first aspect of the invention, there are provided for use in the treatment of migraine and other vascular headaches a tropyl or pseudotropyl benzoate derivative of the following general Formula I:—

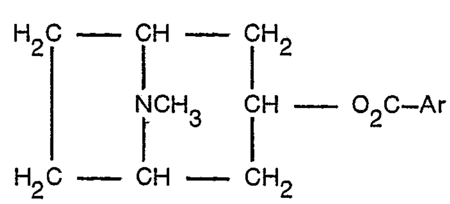

wherein:—

Ar represents

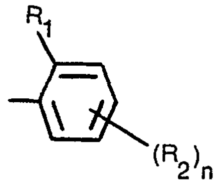

or

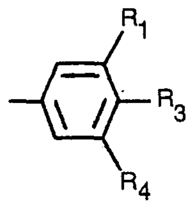

*n* represents 0 or an integer from 1 to 3;

$R_1$ represents $C_1$—$C_4$ alkyl;

$R_2$ represents $C_1$—$C_4$ alkyl, halogen or $C_1$—$C_4$ alkoxy, provided that $R_2$ is the same as $R_1$ when *n* is 2 or 3; and

$R_3$ and $R_4$ independently represent hydrogen, $C_1$—$C_4$ alkyl, $C_1$—$C_4$ alkoxy or halogen provided that for tropyl derivatives $R_3$ is alkyl and $R_4$ is hydrogen,

or a pharmaceutically accetable salt thereof.

According to a second aspect of the invention there are provided pharmaceutical compositions in unit dose form for the effective relief of migraine comprising a compound of general Formula I in admixture or otherwise associated with a pharmaceutically acceptable diluent or carrier and containing 0.5 to 100 mg per unit dose. Usually, said compositions will contain 1 to 50 mg, especially 3 to 30 mg, per unit dose.

According to a third aspect of the invention, there are provided *per se* all compounds of Formula I except tropyl-2-methylbenzoate.

Migraine is treated by administering to a patient suffering migraine, an effective migraine relieving amount of a compound of Formula I. Said amount usually will be in the range 0.01 mg/kg to 10 mg/kg, especially 0.03 mg/kg to 3.0 mg/kg. It is also contemplated that the compounds of Formula I can be used in the prophylaxis of migraine by administering to a patient at risk of migraine an effective migraine-prophylatic amount of the compound.

Detailed Description of the Invention

When the bicyclic ring of a compound of Formula I is in the endo configuration, the compound is a tropyl benzoate and, when it is in the exo configuration, the compound is a pseudotropyl benzoate. Presently, the tropyl benzoates are preferred over the pseudotropyl benzoates.

The compounds of general Formula I have the benzoyloxy moiety substituted by $C_1$—$C_4$ alkyl in at least the 2 or 3 position and optionally in other positions. In particular $R_1$ represents $C_1$—$C_4$ alkyl; $R_2$ represents $C_1$—$C_4$ alkyl, $C_1$—$C_4$ alkoxy or halogen but is the same as $R_1$ when there are two or three $R_2$ substituents; $R_3$ can represent $C_1$—$C_4$ alkyl, $C_1$—$C_4$ alkoxy or halogen instead of hydrogen; $R_4$ can represent $C_1$—$C_4$ alkyl, $C_1$—$C_4$ alkoxy or halogen instead of hydrogen; and *n* is 0 or an integer up to 3 preferably 0, 1 or 2. The tropyl benzoates (but not pseudotropyl benzoates) of Formula I substituted at the 3-position alone, the 3 and 5 positions only, or the 3, 4 and 5 positions only are excluded because they are already the subject of the previously acknowledged co-pending application.

In general Formula I, Ar can represent phenyl substituted as shown in the following Table I:

TABLE I

| Position | 2 | 3 | 4 | 5 | 6 |
|---|---|---|---|---|---|
| | $R_1$ | H | H | H | H |
| | $R_1$ | $R_2$ | H | H | H |
| | $R_1$ | H | $R_2$ | H | H |
| | $R_1$ | H | H | $R_2$ | H |
| | $R_1$ | H | H | H | $R_2$ |
| | $R_1$ | $R_1$ | $R_1$ | H | H |
| | $R_1$ | $R_1$ | H | $R_1$ | H |
| | $R_1$ | $R_1$ | H | H | $R_1$ |
| | $R_1$ | H | $R_1$ | $R_1$ | H |
| | $R_1$ | H | $R_1$ | H | $R_1$ |
| | $R_1$ | $R_1$ | $R_1$ | $R_1$ | H |
| | $R_1$ | $R_1$ | $R_1$ | H | $R_1$ |
| | $R_1$ | $R_1$ | H | $R_1$ | $R_1$ |
| | *H | $R_1$ | H | H | H |
| | H | $R_1$ | $R_3$ | H | H |
| | *H | $R_1$ | H | $R_4$ | H |
| | *H | $R_1$ | $R_3$ | $R_4$ | H |

* pseudotropyl benzoate only

In the table above $R_1$, $R_2$, $R_3$ and $R_4$ are as defined in connection with Formula I except that neither $R_3$ nor $R_4$ represents hydrogen.

Examples of $C_1$—$C_4$ alkyl groups which are represented by $R_1$ and can be represented by $R_2$, $R_3$ and $R_4$ are methyl, ethyl, *n*-propyl, *n*-butyl and *iso*-propyl with methyl and ethyl being preferred.

Examples of $C_1$—$C_4$ alkoxy groups which can be represented by $R_2$, $R_3$ and $R_4$ are methoxy, ethoxy, *n*-propoxy, *n*-butoxy and *iso*-propoxy, with ethoxy and especially, methoxy being preferred.

The halogens which can be represented by $R_2$, $R_3$ and $R_4$ are bromine, chlorine, fluorine and iodine with bromine, fluorine and, especially, chlorine being preferred.

One preferred class of compounds of the invention are those pseudotropyl benzoates and tropyl benzoates of Formula I in which Ar represents:—

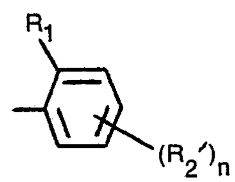

wherein *n* is 0 or an integer from 1 to 3, $R_1$ represents $C_1$—$C_4$ alkyl, and $R_2'$ represents $C_1$—$C_4$ alkyl or chlorine provided that $R_2'$ is the same as $R_1$ when *n* is 2 or 3.

Another preferred class of compounds of the invention are those pseudotropyl benzoates and tropyl benzoates of Formula I in which Ar represents:

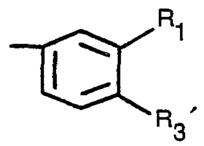

wherein $R_1$ and $R_3'$ independently represent $C_1$—$C_4$ alkyl.

Yet another preferred class of compounds are those pseudotropyl benzoates of Formula I in which Ar represents:—

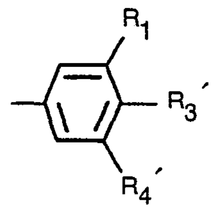

wherein $R_1$ and $R_3'$ independently represent $C_1$—$C_4$ alkyl.

Yet another preferred class of compounds are those pseudotropyl benzoates of Formula I in which Ar represents:—
wherein $R_1$ represents $C_1$—$C_4$ alkyl and $R_3'$ and $R_4'$ independently represent hydrogen or $C_1$—$C_4$ alkyl.

In a presently particularly preferred embodiment of the invention, the compounds are those pseudotropyl benzoates and, preferably, tropyl benzoates of Formula I, in which Ar presents phenyl substituted only by $C_1$—$C_4$ alkyl, especially methyl, at the 2; 2, 3; 2, 4; 2, 5; or 3, 4 positions. The said especially preferred methyl-substituted compounds are the following:—

tropyl-2-methylbenzoate,
tropyl-2,3-dimethylbenzoate,
tropyl-2,4-dimethylbenzoate,
tropyl-2,5-dimethylbenzoate,
tropyl-3,4-dimethylbenzoate,
pseudotropyl-2-methylbenzoate,
pseudotropyl-2,3-dimethylbenzoate,
pseudotropyl-2,4-dimethylbenzoate,
pseudotropyl-2,5-dimethylbenzoate,
pseudotropyl-3,4-dimethylbenzoate.

In another presently especially preferred embodiment of the invention, the compounds are those pseudotropyl benzoates of Formula I in which Ar represents phenyl substituted only by $C_1$—$C_4$ alkyl, especially methyl, at the 3; or 3, 5; or 3, 4, 5 positions. The said especially preferred methyl-substituted compounds are the following:—

pseudotropyl-3-methylbenzoate,
pseudotropyl-3,5-dimethylbenzoate,
pseudotropyl-3,4,5-dimethylbenzoate.

In addition to the preferred compounds specified above, the following are illustrative compounds of Formula I:—

tropyl 2-methyl-3-chlorobenzoate;
tropyl 2-methyl-4-chlorobenzoate;
tropyl 2-methyl-5-chlorobenzoate;
tropyl 2,3,5-trimethylbenzoate;
tropyl 2,3,4,5-tetramethylbenzoate;
tropyl 2-ethylbenzoate;
tropyl 3,4-diethylbenzoate;
tropyl 2-methyl-4-methoxybenzoate;
pseudotropyl 2,3,5-trimethylbenzoate;
pseudotropyl 2-methyl-4-chlorobenzoate;

The compounds of Formula I block the M-receptors for 5-hydroxytryptamine (5-HT) on afferent sensory neurones, certain of which subserve the transmission of pain. As explained above, the blocking of such M-receptors is believed to be a mechanism by which the symptoms of migraine can be relieved. Accordingly, the compounds of Formula I are useful in the treatment of migraine when administered in amounts sufficient to effectively block the said M-receptors.

The activity of the compounds against 5-HT can be assessed by determining their $pA_2$ values in the isolated rabbit heart as described by Fozard *et al.* Europ. J. Pharmacol. *59* 195—210 (1979). In the method described the molar concentration of antagonist which reduces the effects of twice the ED50 of 5-HT to that of the ED50 in the absence of antagonist is determined. The $pA_2$ value is the negative logarithm of said molar concentrations. In general terms, the higher the $pA_2$ value the more potent is the compound.

The $pA_2$ values of some representative compounds of Formula I are given in the following Table II:—

TABLE II

| Compound | $pA_2$ 5-HT |
|---|---|
| tropyl 2-methylbenzoate | 8.1 |
| tropyl 3,4-dimethylbenzoate | 8.2 |
| tropyl 2,5-dimethylbenzoate | 8.6 |
| tropyl 2,4-dimethylbenzoate | 8.8 |
| pseudotropyl 3,5-dimethylbenzoate | 8.9 |

The $pA_2$ values we have measured of some related compounds to those of the invention are given in the following Table III for comparative purposes.

TABLE III

| Compound | $pA_2$ 5-HT |
|---|---|
| pseudotropyl-3,4,5-trimethoxybenzoate | 5.7 |
| pseudotropyl-3,5-dimethoxybenzoate | 6.6 |
| pseudotropyl-4-chlorobenzoate | 6.7 |
| pseudotropyl benzoate | 7.0 |
| tropyl 4-chlorobenzoate | 7.0 |
| tropyl 3,4-dimethoxybenzoate | 7.2 |
| tropyl benzoate | 7.2 |
| pseudotropyl-3,4-dichlorobenzoate | 7.5 |
| tropyl 4-methylbenzoate | 7.8 |
| tropyl 3-methylbenzoate | 8.1 |
| tropyl 3,5-dimethoxybenzoate | 8.4 |
| tropyl 3,4,5-trimethoxybenzoate | 8.5 |
| tropyl 3-chlorobenzoate | 8.6 |
| tropyl 3,5-dimethylbenzoate | 9.0 |
| tropyl 3,5-dichlorobenzoate | 9.3 |

It will be noted *inter alia* from Tables II and III that the compounds of Formula I show in this test a potency as 5-HT M-receptor antagonists at least an order greater than that of tropylbenzoate or pseudotropyl benzoate.

The activity of the compounds against 5-HT can be assessed *in vivo* by measurement of the effect of the compound on the Von Bezold-Jarisch Reflex induced by 5-HT injected intravenously into the rat (see Paintal A.S., Physiol. Rev. *53* 159—227, 1973). The transient cardiac slowing arises from an increased efferent vagus activity arising from stimulation by 5-HT of sensory afferent fibres in and around the heart.

The compounds of Formula I are believed to be highly selective in their action against 5-HT M-receptor. Their potency against other 5-HT receptors and other spasmogens, in particular oxytocin, acetylcholine, histamine and calcium, is believed to be at least two orders lower than that against 5-HT M-receptors. Accordingly, their use in the treatment of migraine should be without any side effects.

The compounds of Formula I can be administered in various manners to achieve the desired effect. The compounds can be administered alone or in the form of pharmaceutical preparations to the patient being treated either orally or parenterally, for example, subcutaneously or intravenously. The amount of compound administered will vary and can be any effective migraine-relieving amount. Depending upon the patient and the mode of administration, the quantity of compound administered may vary over a wide range to provide from about 0.01 mg/kg to about 10 mg/kg, usually 0.03 to 3.0 mg/kg, of body weight of the patient per dose. Unit doses of these compounds can contain, for example, from about 0.5 mg to 100 mg, usually 1 to 50 mg and preferably 3 to 30 mg, of the compound and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

In the composition aspect of the invention there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known *per se.*

The formulations of the invention may be adapted for enteral or parenteral use and may be administered to the patient in the form of tablets, capsules, suppositories, solutions, suspensions or the like.

In the specific examples included hereinbelow illustrative examples of suitable pharmaceutical formulations are described.

The derivatives of Formula I can be used in migraine therapy with antimigraine drugs having different modes of action. Such drugs include those used prophylactically, such as barbiturates, diazepam, chlorpromazine, amitriptyline, propranolol, methysergide, pizotifen, cyproheptadine, dihydroergotamine, and clonidine, and those used in the acute attack, such as vasoconstrictor agents, e.g. ergotamine and dihdyroergotamine, analgaesic/ antiinflammatory agents, e.g. aspirin, paracetamol and indomethacin, or anti-nauseants, e.g. cyclizine, metoclopramide, and triethylperazine (see Fozard, J. R. J. Pharm. Pharmacol. *27*, 297—321 (1975); Saper, J. R., J. Amer. Med. Assoc, *239*, 480—484 (1978); Fozard, J.R., supra.). As an example, compounds of general Formula 1 would be beneficial in combination with aspirin 300—1200 mg or methysergide, 2—6 mg given daily.

The compounds of general Formula I can be prepared in manner known *per se* from tropine or pseudotropine and an acid halide of the following general Formula IV:—

XOC—Ar    Formula IV

wherein Ar is as defined in connection with Formula I, and X represents halogen, especially chlorine.

When preparing tropyl benzoate derivatives, the reaction usually will be carried out in the absence of a solvent by heating, at for example a temperature in the range 140° to 160°C, the acid halide with a hydrohalide salt of tropine whilst stirring. Hydrogen halide is evolved and the mixture first becomes liquid but subsequently becomes solid. Heating is continued for about 15 mins after solidification and the mixture is then cooled and added to water. The product is the hydrohalide of the compound of Formula I and the free base can be obtained by addition of aqueous base, such as sodium or potassium carbonate, which does not hydrolyse the ester, to render the aqueous product solution alkaline and subsequent extraction of the free base with a suitable organic solvent such as, for example, diethylether, ethylacetate and methylene chloride. The organic solution is subsequently evaporated and the residue recrystallized from, for example, aqueous methanol.

When preparing pseudotropyl benzoate derivatives, the reaction usually will be carried out by stirring the acid halide and pseudotropine in an aprotic solvent, preferably methylene chloride or acetonitrile, at ambient temperature. The solvent is evaporated off, usually under reduced pressure, after about 2 to 16 hours and water added to the residue, followed by aqueous base, such as sodium or potassium carbonate, which does not hydrolyse the ester, to render the aqueous product solution alkaline. Subsequently desired free base is extracted with a suitable organic solvent such as, for example, diethyl ether, ethylacetate and methylene chloride. The organic solution is then washed with water to remove excess pseudotropine and dried. The organic solvent is evaporated off and the free base recrystallized from, for example, aqueous

methanol. Alternatively, the crude free base can be converted into an acid addition salt, preferably hydrochloride, by addition of an ethereal solution of the acid. The acid salt is recrystallized from, for example ethanol or isopropanol.

As mentioned previously, the compounds of Formula I can be used in the form of their pharmaceutically acceptable acid addition salts.

The pharmaceutically acceptable acid addition salts can be non-toxic addition salts with suitable acids, such as those with inorganic acids, for example hydrochloric, hydrobromic, nitric, sulfuric or phosphoric acids, or with organic acids, such as organic carboxylic acids, or example, glycollic, maleic, hydroxymaleic, malic, tartaric, citric, salicylic, *o*-acetyloxybenzoic, nicotonic or isonicotinic, or organic sulphonic acids, for example methane sulphonic, ethane sulphonic, 2-hydroxyethane sulphonic, toluene-*p*-sulphonic, or naphthalene-2-sulphonic acids.

Apart from pharmaceutically acceptable acid addition salts, other acid addition salts, such as for example, those with picric or oxalic acid, may be serve as intermediates in the purification of the compounds or in the preparation of other, for example, pharmaceutically acceptable, acid addition salts, or are useful for identification or characterisation of the bases.

An acid addition salt may be converted into the free compound according to known methods, for example, by treating it with a base, such as with a metal hydroxide or alkoxide, for example an alkali or alkaline earth metal hydroxide, for example, lithium hydroxide, sodium hydroxide, potassium hydroxide or calcium hydroxide; with a metal carbonate, such as an alkali metal or an alkaline earth metal carbonate or hydrogen carbonate, for example, sodium, potassium or calcium carbonate or hydrogen carbonate; with trialkylamine; or with an anion exchange resin.

An acid addition salt may also be converted into another acid addition salt according to known methods; for example, a salt with an inorganic acid may be treated with a metal salt, for example a sodium, barium or silver salt, or an acid in a suitable diluent, in which a resulting inorganic salt is insoluble and is thus removed from the reaction medium. Acid addition salt may also be converted into another acid addition salt by treatment with an anion exchange preparation.

The invention is illustrated in the following non-limiting Examples.

### Example 1

Tropyl-3,4-dimethylbenzoate (i.e. 3,4-dimethylbenzoic acid endo-8-methyl-8-azabicyclo[3,2,1]oct-3-yl ester (formula I, endo, Ar = 3,4-dimethylphenyl)

A stirred mixture of tropine hydrochloride (1.76 g) and 3,4-dimethyl benzoyl chloride (1.65 g) is heated at 130—140°C for 30 minutes during which time the mixture liquifies, evolves hydrogen chloride gas and resolidifies. A solution of the cooled solid in water is basified with a solution of potassium carbonate and the base extracted with ethyl acetate. The ethyl acetate solution is washed several times with water, dried over magnesium sulphate, and evaporated to give the free base which is converted to the hydrochloride by the addition of ethereal hydrogen chloride. Recrystallization of the precipitated solid from ethanol gives crystals of tropyl 3,4-dimethylbenzoate hydrochloride mp. 270—271°C.

The following compounds are prepared by the same method:—

tropyl 2-methylbenzoate hydrochloride m.p. 262—3°C;
tropyl 2,4-dimethylbenzoate hydrochloride m.p. 256°C;
tropyl 2,5-dimethylbenzoate hydrochloride m.p. 269.5—270°C.

### Example 2

Pseudotropyl 3,5-dimethylbenzoate (i.e. 3,5-dimethyl-benzoic acid exo-8-methyl-8-azabicyclo[3,2,1]oct-3-yl ester) (formula I, exo, Ar = 3,5-dimethylphenyl)

Pseudotropine (1.41 g) and 3,5-dimethylbenzoylchloride (1.65 g) in methylene chloride (50 ml) is stirred at ambient temperature for 3 hours. The solvent is evaporated off under reduced pressure and the residue treated with water (50 ml) and saturated aqueous potassium carbonate solution (5 ml) is added. The liberated oil is extracted into diethylether, the ethereal solution washed several times with water and then dried over anhydrous magnesium sulfate. Distillation of the dried ethereal solution gives a residue which is treated with anhydrous diethyl ether and ethereal hydrogen chloride. Crystallization of the precipitate from ethanol affords pseudotropyl-3,5-dimethylbenzoate hydrochloride mp 245°C.

In the following Examples relating to pharmaceutical compositions, the term "active compound" is used to indicate the compound tropyl-2,4-dimethylbenzoate. This compound may be replaced in these compositions by any other compound of Formula I, for example by pseudotropyl-3,5-dimethylbenzoate. Adjustments in the amount of medicament may be necessary or desirable depending upon the degrees of activity of the medicament as is well known in the art.

### Example 3

An illustrative composition for hard gelatin capsules is as follows:—

(a) active compound 5 mg
(b) talc 5 mg
(c) lactose 90 mg

The formulation is prepared by passing the dry powders of (a) and (b) through a fine mesh screen and mixing them well. The powder is then filled into hard gelatin capsules at a net fill of 100 mg per capsule.

Example 4

An illustrative composition for tablets is as follows:—

(a) active compound 5 mg
(b) starch 43 mg
(c) lactose 50 mg
(d) magnesium stearate 2 mg

The granulation obtained upon mixing the lactose with the compound (a) and part of the starch and granulating with starch paste is dried, screened, and mixed with the magnesium stearate. The mixture is compressed into tables weighing 100 mg each.

Example 5

An illustrative composition for an injectable suspension is the following 1 ml ampule for an intramuscular injection:—

| | Weight per cent |
|---|---|
| (a) active compound | 0.01 |
| (b) polyvinylpyrrolidone | 0.5 |
| (c) lecithin | 0.25 |
| (d) water for injection to make | 100.0 |

The material (a)—(d) are mixed, homogenized, and filled into 1 ml ampules which are sealed and autoclaved 20 minutes at 121°C. Each ampule contains 1.0 mg per ml of compound (a).

Example 6

| | mg/suppository |
|---|---|
| Active Compound | 5 |
| Oil of Theobroma (cocoa butter) | 995 |

The medicament is powdered and passed through a B.S. No. 100 Sieve and triturated with molten oil of theobroma at 45°C to form a smooth suspension. The mixture is well stirred and poured into moulds each of nominal 1G capacity, to produce suppositories.

**Claims for the Contracting States: BE CH DE FR IT LI LU NL SE**

1. A pharmaceutical composition in unit dose form for the treatment of migraine comprising, in admixture or otherwise associated with a pharmaceutically acceptable diluent or carrier, an amount of 0.5 to 100 mg per unit dose of a tropyl or pseudotropyl benzoate derivative of the following general Formula I:—

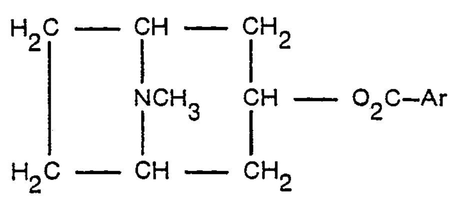

wherein:—

Ar represents

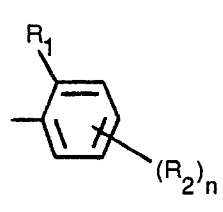

or

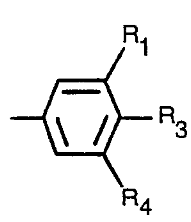

$n$ represents 0 or an integer from 1 to 3

$R_1$ represents $C_1$—$C_4$ alkyl;

$R_2$ represents $C_1$—$C_4$ alkyl, $C_1$—$C_4$ alkoxy or halogen provided that $R_2$ is the same as $R_1$ when $n$ is 2 or 3; and

$R_3$ and $R_4$ independently represent hydrogen, $C_1$—$C_4$ alkyl, $C_1$—$C_4$ alkoxy, or halogen provided that for tropyl derivatives $R_3$ is alkyl and $R_4$ is hydrogen;

or a pharmaceutically acceptable salt thereof.

2. A tropyl or pseudotropyl benzoate derivative of the following general Formula I:—

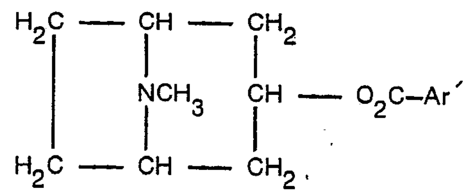

wherein:—

Ar′ represents

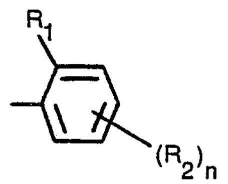

or

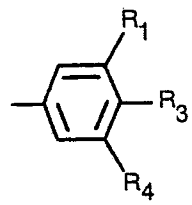

$n$ represents 0 or an integer from 1 to 3

$R_1$ represents $C_1$—$C_4$ alkyl provided that $R_1$ is not methyl when $n$ is 0;

$R_2$ represents $C_1$—$C_4$ alkyl, $C_1$—$C_4$ alkoxy or halogen, provided that $R_2$ is the same as $R_1$ when $n$ is 2 or 3; and

$R_3$ and $R_4$ independently represent hydrogen, $C_1$—$C_4$ alkyl, $C_1$—$C_4$ alkoxy, or halogen, provided that for tropyl derivatives $R_3$ is alkyl and $R_4$ is hydrogen;

or a pharmaceutically acceptable salt thereof.

3. A compound as claimed in Claim 2 wherein Ar′ represents:—

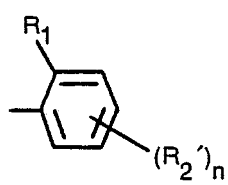

wherein $n$ is 0 or an integer from 1 to 3, $R^1$ represents $C_1$—$C_4$ alkyl and $R_2'$ represents $C_1$—$C_4$ alkyl or chlorine, provided that $R_1$ is not methyl when $n$ is 0 and that $R_2'$ is the same as $R_1$ when $n$ is 2 or 3, or a pharmaceutically acceptable salt thereof.

4. A compound as claimed in Claim 3 wherein $R_1$ and $R_2'$ both represent methyl, or a pharmaceutically acceptable salt thereof.

5. A compound as claimed in Claim 3 wherein $n$ is 1 and the phenyl group is substituted in the 2,3; 2,4; or 2,5 positions, or a pharmaceutically acceptable salt thereof.

6. A compound as claimed in Claim 3 which is a tropyl benzoate derivative, or a pharmaceutically acceptable salt thereof.

7. A compound as claimed in Claim 3 which is tropyl 2,4-dimethylbenzoate, tropyl 2,5-dimethylbenzoate, or a pharmaceutically acceptable salt thereof.

8. A compound as claimed in Claim 2 which is a tropyl benzoate derivative of Formula I in which Ar′ represents:—

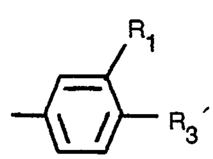

wherein $R_1$ and $R_3'$ independently represent $C_1$—$C_4$ alkyl groups, or a pharmaceutically acceptable salt thereof.

9. A compound as claimed in Claim 8 which is tropyl-3,4-dimethylbenzoate, or a pharmaceutically acceptable salt thereof.

10. A compound as claimed in Claim 2 which is a pseudotropyl benzoate of Formula I in which Ar' represents:—

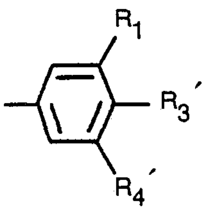

wherein $R_1$ represents $C_1$—$C_4$ alkyl, and $R_3'$ and $R_4'$ independently represent hydrogen or $C_1$—$C_4$ alkyl, or a pharmaceutically acceptable salt thereof.

11. A compound as claimed in Claim 10 wherein $R_1$ represents methyl, and $R_3'$ and $R_4'$ independently represent hydrogen or methyl, or a pharmaceutically acceptable salt thereof.

12. A compound as claimed in Claim 11 which is pseudotropyl 3,5-dimethyl benzoate, or a pharmaceutically acceptable salt thereof.

13. A tropyl or pseudotropyl benzoate derivative as claimed in any one of Claims 2 to 12 or a pharmaceutically acceptable salt thereof for use in the treatment of migraine.

14. A composition as claimed in Claim 1 wherein the compound of general Formula I is as claimed in any one of Claim 2 to 12.

15. A composition as claimed in Claim 1 wherein the compound of general Formula I is tropyl-2-methylbenzoate.

16. The use of a compound as claimed in any one of Claims 2 to 12 or a pharmaceutically acceptable salt thereof for the manufacture of a medicament for the treatment of migraine.

17. Tropyl-2-methyl benzoate or a pharmaceutically acceptable salt thereof for use in the treatment of migraine.

18. The use of tropyl-2-methyl benzoate or a pharmaceutically acceptable salt thereof for the manufacture of a medicament for the treatment of migraine.

**Claims for the Contracting State: AT**

1. An analogous process for preparing a tropyl or pseudotropyl benzoate derivative of the following general Formula I:—

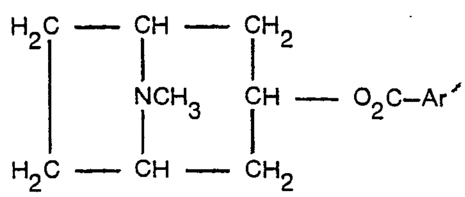

(I)

wherein:—

Ar' represents

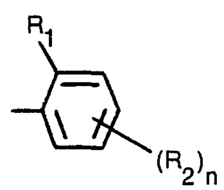

or

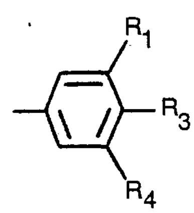

*n* represents 0 or an integer from 1 to 3

$R_1$ represents $C_1$—$C_4$ alkyl provided that $R_1$ is not methyl when *n* is 0;

$R_2$ represents $C_1$—$C_4$ alkyl, $C_1$—$C_4$ alkoxy or halogen, provided that $R_2$ is the same as $R_1$ when *n* is 2 or 3; and

$R_3$ and $R_4$ independently represent hydrogen, $C_1$—$C_4$ alkyl, $C_1$—$C_4$ alkoxy, or halogen, provided that for tropyl derivatives $R_3$ is alkyl and $R_4$ is hydrogen; or a pharmaceutically acceptable salt thereof which comprises the reaction in manner known *per se* of tropine or pseudotropine and the corresponding acid halide of the following general Formula IV:—

$$XOC—Ar' \qquad \text{Formula IV}$$

wherein Ar' is as defined above and X represents halogen.

2. A process as claimed in Claim 1 wherein Ar' represents:—

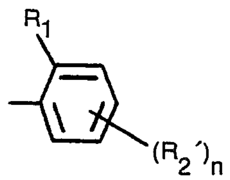

wherein $n$ is 0 or an integer from 1 to 3; $R_1$ represents $C_1$—$C_4$ alkyl and $R_2$ represents $C_1$—$C_4$ alkyl or chlorine, provided that $R_1$ is not methyl when $n$ is 0 and that $R_2'$ is the same as $R_1$ when $n$ is 2 or 3.

3. A process as claimed in Claim 2 wherein $R_1$ and $R_2'$ both represent methyl.

4. A process as claimed in Claim 2 wherein $n$ is 1 and Ar' is phenyl substituted in the 2,3; 2,4; or 2,5 positions.

5. A process as claimed in Claim 2 wherein tropine is reacted with the acid halide to give the corresponding tropyl benzoate of Formula I.

6. A process as claimed in Claim 1 wherein tropine is reacted with an acid halide of the following Formula IV*a*:—

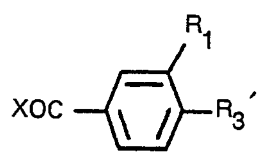

(IVa)

wherein $R_1$ and $R_3'$ independently represent $C_1$—$C_4$ alkyl groups and X represents halogen, to give the corresponding tropyl benzoate of Formula I.

7. A process as claimed in Claim 6 wherein $R_1$ and $R_3'$ are both methyl.

8. A process as claimed in Claim 1 wherein pseudotropine is reacted with an acid halide of the following Formula IV*b*:—

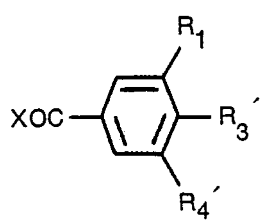

(IVb)

wherein $R_1$ represents $C_1$—$C_4$ alkyl, $R_3'$ and $R_4'$ independently represent hydrogen or $C_1$—$C_4$ alkyl, and X represents halogen to give the corresponding pseudotropyl benzoate of Formula I.

9. A process as claimed in Claim 8 wherein $R_1$ represents methyl, and $R_3'$ and $R_4'$ independently represent hydrogen or methyl.

10. A method of preparing a pharmaceutical composition wherein a pharmacologically active ingredient is mixed or otherwise associated with a pharmaceutically acceptable diluent or carrier, characterised in that the active ingredient is a pseudotropyl or tropyl benzoate derivative as defined in any one of Claims 1 to 9 or a pharmaceutically acceptable salt thereof.

**Patentansprüche für die Vertragsstaaten: BE CH DE FR IT LI LU NL SE**

1. Arzneimittel in Dosiseinheitsform zur Migränebehandlung, enthaltend, im Gemisch oder in anderer Weise verbunden mit einem pharmakologisch verträglichen Verdünnungsmittel oder Träger, eine Menge von 0,5 bis 100 mg pro Dosiseinheit eines Tropyl- oder Pseudotropylbenzoat-Derivats der folgenden allgemeinen Formel I:

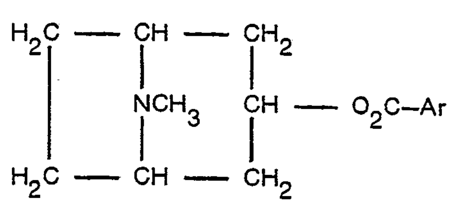

(I)

in der Ar

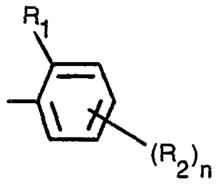

oder

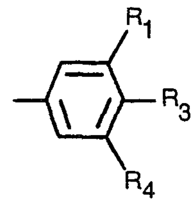

darstellt,

n 0 oder eine ganze Zahl von 1 bis 3 bedeutet,

$R_1$ einen $C_1$—$C_4$-Alkylrest darstellt,

$R_2$ einen $C_1$—$C_4$-Alkylrest, einen $C_1$—$C_4$-Alkoxyrest oder ein Halogenatom bedeutet, mit der Maßgabe, daß der Rest $R_2$ die gleiche Bedeutung wie der Rest $R_1$ hat, wenn n den Wert 2 oder 3 hat, und

$R_3$ und $R_4$ unabhängig voneinander ein Wasserstoffatom, einen $C_1$—$C_4$-Alkylrest, einen $C_1$—$C_4$ Alkoxyrest, oder ein Halogenatom bedeuten, mit der Maßgabe, daß bei Tropyl-Derivaten $R_3$ einen Alkylrest und $R_4$ ein Wasserstoffatom bedeutet; oder ein pharmakologisch verträgliches Salz davon.

2. Tropyl- oder Pseudotropylbenzoat-Derivat der folgenden allgemeinen Formel I

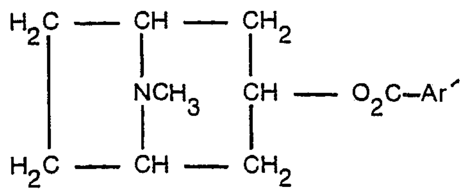

(I)

in der Ar'

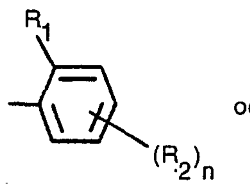

oder

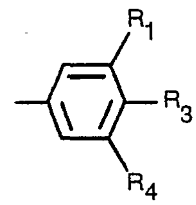

darstellt,

n 0 oder eine ganze Zahl von 1 bis 3 bedeutet,

$R_1$ einen $C_1$—$C_4$-Alkylrest darstellt, mit der Maßgabe, daß der Rest $R_1$ keine Methylgruppe ist, wenn n den Wert 0 hat,

$R_2$ einen $C_1$—$C_4$-Alkylrest, einen $C_1$—$C_4$-Alkoxyrest oder ein Halogenatom bedeutet, mit der Maßgabe, daß $R_2$ die gleiche Bedeutung wie $R_1$ hat, wenn n den Wert 2 oder 3 hat, und $R_3$ und $R_4$ unabhängig voneinander ein Wasserstoffatom, einen $C_1$—$C_4$-Alkylrest, einen $C_1$—$C_4$-Alkoxyrest oder ein Halogenatom bedeuten, mit der Maßgabe, daß bei Tropylderivaten $R_3$ einen Alkylrest und $R_4$ ein Wasserstoffatom bedeutet; oder ein pharmakologisch verträgliches Salz davon.

3. Verbindung nach Anspruch 2, in der Ar' die folgende Bedeutung hat:

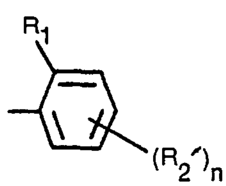

wobei n 0 oder eine ganze Zahl von 1 bis 3 bedeutet, $R_1$ einen $C_1$—$C_4$-Alkylrest darstellt und $R_2'$ einen $C_1$—$C_4$-Alkylrest oder ein Chloratom bedeutet, mit der Maßgabe, daß der Rest $R_1$ keine Methylgruppe ist, wenn n den Wert 0 hat und daß der Rest $R_2'$ die gleiche Bedeutung wie $R_1$ hat, wenn n den Wert 2 oder 3 hat; oder ein pharmakologisch verträgliches Salz davon.

4. Verbindung nach Anspruch 3, in der $R_1$ und $R_2'$ jeweils eine Methylgruppe bedeuten, oder ein pharmakologisch verträgliches Salz davon.

5. Verbindung nach Anspruch 3, in der n den Wert 1 hat und die Phenylgruppe in den 2,3-, 2,4- oder 2,5-Stellungen substituiert ist, oder ein pharmakologisch verträgliches Salz davon.

6. Verbindung nach Anspruch 3, nämlich ein Tropylbenzoat-Derivat oder ein pharmakologisch verträgliches Salz davon.

7. Verbindung nach Anspruch 3, nämlich Tropyl-2,4-dimethylbenzoat, Tropyl-2,5-dimethylbenzoat oder deren pharmakologisch verträgliche Salze.

8. Verbindung nach Anspruch 2, nämlich ein Tropylbenzoat-Derivat der allgemeinen Formel I, in der Ar' die folgende Bedeutung hat:

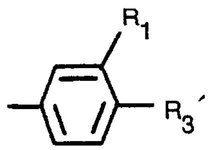

wobei $R_1$ und $R_3'$ unabhängig voneinander $C_1$—$C_4$-Alkylreste bedeuten, oder ein pharmakologisch verträgliches Salz davon.

9. Verbindung nach Anspruch 8, nämlich Tropyl-3,4-dimethylbenzoat oder ein pharmakologisch verträgliches Salz davon.

10. Verbindung nach Anspruch 2, nämlich ein Pseudotropylbenzoat der allgemeinen Formel I, in der Ar' die folgende Bedeutung hat:

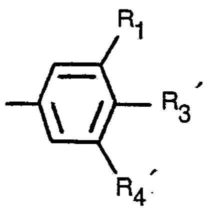

wobei $R_1$ einen $C_1$—$C_4$-Alkylrest bedeutet und $R_3'$ und $R_4'$ unabhängig voneinander ein Wasserstoffatom oder einen $C_1$—$C_4$-Alkylrest darstellen, oder ein pharmakologisch verträgliches Salz davon.

11. Verbindung nach Anspruch 10, in der $R_1$ eine Methylgruppe bedeutet, und $R_3'$ und $R_4'$ unabhängig voneinander ein Wasserstoffatom oder eine Methylgruppe bedeuten, oder ein pharmakologisch verträgliches Salz davon.

12. Verbindung nach Anspruch 11, nämlich Pseudotropyl-3,5-dimethylbenzoat oder ein pharmakologisch verträgliches Salz davon.

13. Tropyl- oder Pseudotropylbenzoat-Derivat nach jedem der Ansprüche 2 bis 12 oder ein pharmakologisch verträgliches Salz davon zur Verwendung in der Migränebehandlung.

14. Mittel nach Anspruch 1 mit einer Verbindung der allgemeinen Formel I nach jedem der Ansprüche 2 bis 12.

15. Mittel nach Anspruch 1, in dem die Verbindung der allgemeinen Formel I Tropyl-2-methylbenzoat ist.

16. Verwendung einer Verbindung nach jedem der Ansprüche 2 bis 12 oder eines pharmakologisch verträglichen Salzes davon zur Herstellung eines Arzneistoffes zur Migränebehandlung.

17. Tropyl-2-methylbenzoat oder ein pharmakologisch verträgliches Salz davon zur Verwendung in der Migränebehandlung.

18. Verwendung von Tropyl-2-methylbenzoat oder eines pharmakologisch verträglichen Salzes davon für die Herstellung eines Arzneistoffes zur Migränebehandlung.

**Patentansprüche für den Vertragsstaat: AT**

1. Analogverfahren zur Herstellung eines Tropyl- oder Pseudotropylbenzoat-Derivats der folgenden allgemeinen Formel I:

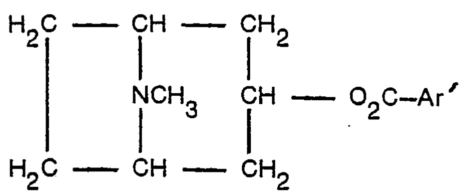

in der Ar'

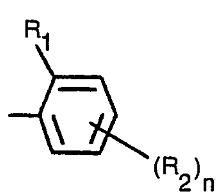

oder

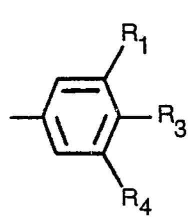

darstellt, n 0 oder eine ganze Zahl von 1 bis 3 bedeutet,

$R_1$ einen $C_1$—$C_4$-Alkylrest darstellt, mit der Maßgabe, daß der Rest $R_1$ keine Methylgruppe ist, wenn n den Wert 0 hat,

$R_2$ einen $C_1$—$C_4$-Alkylrest, $C_1$—$C_4$-Alkoxyrest oder ein Halogenatom bedeutet, mit der Maßgabe, daß der Rest $R_2$ die gleiche Bedeutung wie $R_1$ hat, wenn n den Wert 2 oder 3 hat, und $R_3$ und $R_4$ unabhängig voneinander ein Wasserstoffatom, einen $C_1$—$C_4$-Alkylrest, einen $C_1$—$C_4$-Alkoxyrest oder ein Halogenatom bedeuten, mit der Maßgabe, daß bei Tropylderivaten $R_3$ einen Alkylrest und $R_4$ ein Wasserstoffatom bedeutet;

oder eines pharmakologisch verträglichen Salzes davon, gekennzeichnet durch die an sich bekannte Umsetzung von Tropin oder Pseudotropin und des entsprechenden Säurehalogenids der folgenden allgemeinen Formel IV:

$$XOC—Ar' \qquad (IV)$$

in der Ar' die vorstehend angegebene Bedeutung hat und X ein Halogenatom darstellt.

2. Verfahren nach Anspruch 1, in dem Ar' die folgende Bedeutung hat:

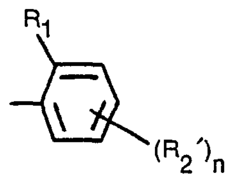

wobei n 0 oder eine ganze Zahl von 1 bis 3 bedeutet, $R_1$ einen $C_1$—$C_4$-Alkylrest darstellt und $R_2$ einen $C_1$—$C_4$-Alkylrest oder ein Chloratom bedeutet, mit der Maßgabe, daß der Rest $R_1$ keine Methylgruppe ist, wenn n den Wert 0 hat, und daß der Rest $R_2'$ die gleiche Bedeutung wie $R_1$ hat, wenn n den Wert 2 oder 3 hat.

3. Verfahren nach Anspruch 2, in dem $R_1$ und $R_2'$ jeweils eine Methylgruppe bedeuten.

4. Verfahren nach Anspruch 2, in dem n den Wert 1 hat und Ar' eine in den 2,3-, 2,4- oder 2,5-Stellungen substituierte Phenylgruppe bedeutet.

5. Verfahren nach Anspruch 2, in dem Tropin mit dem Säurehalogenid zu dem entsprechenden Tropylbenzoat der allgemeinen Formel I umgesetzt wird.

6. Verfahren nach Anspruch 1, in dem Tropin mit einem Säurehalogenid der allgemeinen Formel IVa:

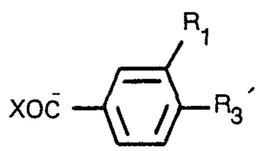

(IVa)

in der $R_1$ und $R_3'$ unabhängig voneinander $C_1$—$C_4$-Alkylreste bedeuten und X ein Halogenatom darstellt, zu dem entsprechenden Tropylbenzoat der allgemeinen Formel I umgesetzt wird.

7. Verfahren nach Anspruch 6, in dem $R_1$ und $R_3'$ jeweils eine Methylgruppe bedeuten.

8. Verfahren nach Anspruch 1, in dem Pseudotropin mit einem Säurehalogenid der allgemeinen Formel IVb:

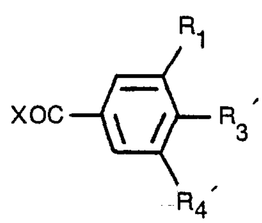

(IVb)

in der $R_1$ einen $C_1$—$C_4$-Alkylrest bedeutet, $R_3'$ und $R_4'$ unabhängig voneinander ein Wasserstoffatom oder einen $C_1$—$C_4$-Alkylrest darstellen und X ein Halogenatom bedeutet, zu dem entsprechenden Pseudotropylbenzoat der allgemeinen Formel I umgesetzt wird.

9. Verfahren nach Anspruch 8, in dem $R_1$ eine Methylgruppe bedeutet und $R_3'$ und $R_4'$ unabhängig voneinander ein Wasserstoffatom oder eine Methylgruppe darstellen.

10. Verfahren zur Herstellung eines Arzneimittels, in dem ein pharmakologisch wirksamer Stoff mit einem pharmakologisch verträglichen Verdünnungsmittel oder Träger gemischt oder in anderer Weise verbunden wird, dadurch gekennzeichnet, daß der Wirkstoff ein Pseudotropyl- oder Troplybenzoat-Derivat nach jedem der Ansprüche 1 bis 9 oder ein pharmakologisch verträgliches Salz davon ist.

**Revendications pour les Etats contractants: BE CH DE FR IT LI LU NL SE**

1. Une composition pharmaceutique sous forme d'une dose unitaire pour le traitement de la migraine comprenant, en mélange ou un association autre avec un diluant ou support convenant en pharmacie, une quantité de 0,5 à 100 mg par dose unitaire d'un dérivé de type benzoate de tropyle ou de pseudotropyle répondant à la formule générale I suivante:

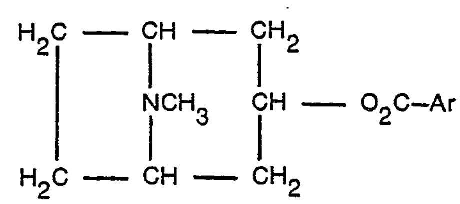

dans laquelle

Ar représente

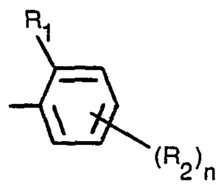

ou

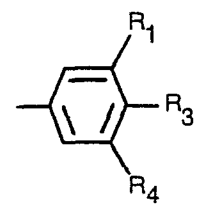

n représente 0 ou un entier de 1 à 3

$R_1$ représente un alkyle en $C_1$—$C_4$

$R_2$ représente un alkyle en $C_1$—$C_4$, un alcoxy en $C_1$—$C_4$ ou un halogène, sous réserve que $R_2$ soit comme $R_1$ lorsque n est 2 ou 3; et

$R_3$ et $R_4$ représentent indépendamment un hydrogène, un alkyle en $C_1$—$C_4$, un alcoxy en $C_1$—$C_4$ ou un halogène, sous réserve que pour les dérivés de tropyle $R_3$ soit un alkyle et $R_4$ soit un hydrogène;

ou un sel convenant en pharmacie correspondant.

2. Un dérivé de type benzoate de tropyle ou de pseudotropyle répondant à la formule générale I suivante:

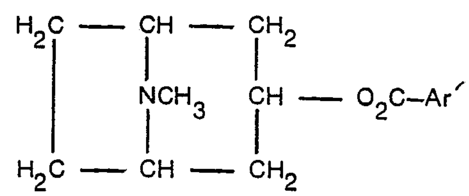

dans laquelle

Ar' représente

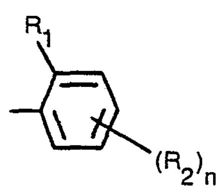

ou

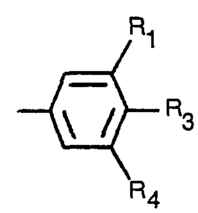

n représente 0 ou un entier de 1 à 3

$R_1$ représente un alkyle en $C_1$—$C_4$, sous réserve que $R_1$ n'est pas un méthyle lorsque n est 0;

$R_2$ représente un alkyle en $C_1$—$C_4$, un alcoxy en $C_1$—$C_4$ ou un halogène, sous réserve que $R_2$ est comme $R_1$ lorsque n est 2 ou 3; et

$R_3$ et $R_4$ représentent indépendamment un hydrogène, un alkyle en $C_1$—$C_4$, un alcoxy en $C_1$—$C_4$ ou un halogène sous réserve que, pour les dérivés de tropyle, $R_3$ est un alkyle et $R_4$ est un hydrogène;

ou un sel convenant en pharmacie correspondant.

3. Un composé comme revendiqué dans la revendication 2, dans lequel Ar' représente:

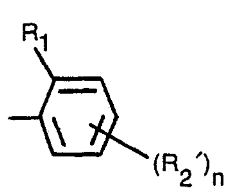

où n est 0 ou un entier de 1 à 3, $R_1$ représente un alkyle en $C_1$—$C_4$ et $R_2'$ représente un alkyle en $C_1$—$C_4$ ou un chlore, sous réserve que $R_1$ n'est pas un méthyle lorsque n est 0 et que $R_2'$ est comme $R_1$ lorsque n est 2 ou 3, ou un sel convenant en pharmacie correspondant.

4. Un composé comme revendiqué dans la revendication 3, dans lequel $R_1$ et $R_2'$ représentent tous deux un méthyle ou un sel acceptable en pharmacie correspondant.

5. Un composé comme revendiqué dans la revendication 3, dans lequel n est 1 et le groupe phényle est substitué dans les positions 2,3; 2,4; ou 2,5 ou un sel convenant en pharmacie correspondant.

6. Un composé comme revendiqué dans la revendication 3, qui est un dérivé de type benzoate de tropyle ou un sel convenant en pharmacie correspondant.

7. Un composé comme revendiqué dans la revendication 3, qui est le 2,4-diméthylbenzoate de tropyle, le 2,5-diméthylbenzoate de tropyle ou un sel convenant en pharmacie correspondant.

8. Un composé comme revendiqué dans la revendication 2, qui est un dérivé de type benzoate de tropyle de formule I dans laquelle Ar' représente:

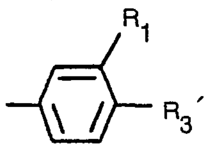

où $R_1$ et $R_3'$ représentent indépendamment des groupes alkyles en $C_1$—$C_4$, ou un sel convenant en pharmacie correspondant.

9. Un composé comme revendiqué dans la revendication 8, qui est le 3,4-diméthylbenzoate de tropyle ou un sel convenant en pharmacie de celui-ci.

10. Un composé comme revendiqué dans la revendication 2, qui est un benzoate de pseudotropyle de formule I dans laquelle Ar' représente:

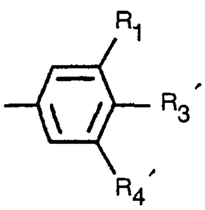

dans laquelle $R_1$ représente un alkyle en $C_1$—$C_4$ et $R_3'$ et $R_4'$ représentent indépendamment un hydrogène ou un alkyle en $C_1$—$C_4$, ou un sel convenant en pharmacie correspondant.

11. Un composé comme revendiqué dans la revendication 10, dans lequel $R_1$ représente un méthyle et $R_3'$ et $R_4'$ représentent indépendamment un hydrogène ou un méthyle ou un sel convenant en pharmacie correspondant.

12. Un composé comme revendiqué dans la revendication 11, qui est le 3,5-diméthylbenzoate de pseudotropyle ou un sel convenant en pharmacie de celui-ci.

13. Un dérivé de type benzoate de tropyle ou de pseudotropyle comme revendiqué dans l'une quelconque des revendications 2 à 12 ou un sel convenant en pharmacie correspondant pour l'emploi dans le traitement de la migraine.

14. Une composition comme revendiqué dans la revendication 1, dans laquelle le composé de formule générale I est comme revendiqué dans l'une quelconque des revendications 2 à 12.

15. Une composition comme revendiqué dans la revendication 1, dans laquelle le composé de formulé générale I est le 2-méthylbenzoate de tropyle.

16. L'emploi d'un composé comme revendiqué dans l'une quelconque des revendications 2 à 12 ou d'un sel convenant en pharmacie correspondant pour la préparation d'un médicament pour le traitement de la migraine.

17. Le 2-méthylbenzoate de tropyle ou un sel convenant en pharmacie de celui-ci pour l'emploi dans le traitement de la migraine.

18. L'emploi du 2-méthylbenzoate de tropyle ou d'un sel convenant en pharmacie de celui-ci pour la préparation d'un médicament pour le traitement de la migraine.

**Revendications pour l'Etat contractant: AT**

1. Un procédé analogue pour la préparation d'un dérivé de type benzoate de tropyle ou de pseudotropyle répondant à la formule générale I suivante:

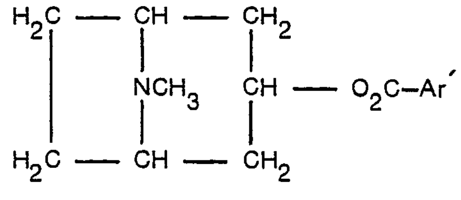

dans laquelle

Ar' représente:

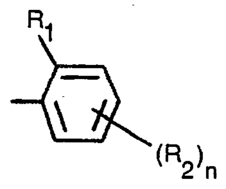

ou

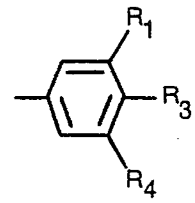

n représente 0 ou un entier de 1 à 3

$R_1$ représente un alkyle en $C_1$—$C_4$, sous réserve que $R_1$ ne soit pas un méthyle lorsque n est 0;

$R_2$ représente un alkyle en $C_1$—$C_4$, un alcoxy en $C_1$—$C_4$ ou un halogène, sous réserve que $R_2$ soit comme $R_1$ lorsque n est 2 ou 3; et

$R_3$ et $R_4$ représentent indépendamment un hydrogène, un alkyle en $C_1$—$C_4$, un alcoxy en $C_1$—$C_4$ ou un halogène, sous réserve que, pour les dérivés de type tropyle, $R_3$ soit un alkyle et $R_4$ soit un hydrogène;

ou un sel convenant en pharmacie correspondant, qui comprend la réaction de façon connue en soi de la tropine ou de la pseudotropine et de l'halogénure d'acide correspondant répondant à la formule générale IV suivante:

XOC—Ar' Formula IV

dans laquelle Ar' est comme défini ci-dessus et X représente un halogène.

2. Un procédé comme revendiqué dans la revendication 1, dans lequel Ar' représente:

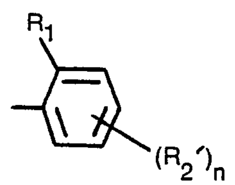

où n est 0 ou un entier de 1 à 3, $R_1$ représente un alkyle en $C_1$—$C_4$ et $R_2$ représente un alkyle en $C_1$—$C_4$ ou un chlore, sous réserve que $R_1$ ne soit pas un méthyle lorsque n est 0 et que $R_2'$ soit comme $R_1$ lorsque n est 2 ou 3.

3. Un procédé comme revendiqué dans la revendication 2, dans lequel $R_1$ et $R_2'$ représentent tous deux un méthyle.

4. Un procédé comme revendiqué dans la revendication 2, dans lequel n est 1 et Ar' est un phényle substitué dans les positions 2,3; 2,4; ou 2,5.

5. Un procédé comme revendiqué dans la revendication 2, dans lequel on fait réagir la tropine avec l'halogénure d'acide pour fournir le benzoate de tropyle correspondant de formule I.

6. Un procédé comme revendiqué dans la revendication 1, dans lequel on fait réagir la tropine avec un halogénure d'acide répondant à la formule IVa suivante:

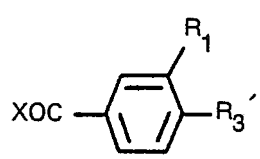

(IVa)

dans laquelle $R_1$ et $R_3'$ représentent indépendamment des groupes alkyles en $C_1$—$C_4$ et X représente un halogène, pour fournir le benzoate de tropyle correspondant de formule I.

7. Un procédé comme revendiqué dans la revendication 6, dans lequel $R_1$ et $R_3'$ sont tous deux un méthyle.

8. Un procédé comme revendiqué dans la revendication 1, dans lequel on fait réagir la pseudotropine avec un halogénure d'acide de formule générale IVb suivante:

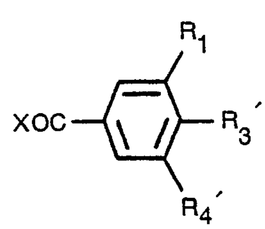

(IVb)

dans laquelle $R_1$ représente un alkyle en $C_1$—$C_4$, $R_3'$ et $R_4'$ représentent indépendamment un hydrogène ou un alkyle en $C_1$—$C_4$ et X représente un halogène, pour fournir le benzoate de pseudotropyle correspondant de formule I.

9. Un procédé comme revendiqué dans la revendication 8, dans lequel $R_1$ représente un méthyle et $R_3'$ et $R_4'$ représentent indépendamment un hydrogène ou un méthyle.

10. Un procédé pour la préparation d'un composition pharmaceutique, dans lequel on mélange ou associe d'autre façon un ingrédient à activité pharmacologique avec un diluant ou support convenant en pharmacie, caractérisé en ce que l'ingrédient actif est un dérivé de type benzoate de pseudotropyle ou de tropyle comme défini dans l'une quelconque des revendications 1 à 9 ou un sel convenant en pharmacie correspondant.